# EUROPEAN PATENT SPECIFICATION

(11) **EP 1 937 250 B1**
(45) Date of publication and mention of the grant of the patent: **19.10.2011**
(21) Application number: 06753874.4
(22) Date of filing: 24.05.2006
(51) Int. Cl.: A61K 31/343, A61P 29/00, A61P 19/02, A61P 19/06, A61P 11/00

(54) **LIGUSTILIDE DERIVATIVES FOR THE TREATMENT OF INFLAMMATORY DISORDERS**
LIGUSTILID-DERIVATE ZUR BEHANDLUNG VON ENTZÜNDUNGSERKRANKUNGEN
DERIVES DE LIGUSTILIDE POUR LE TRAITEMENT DE MALADIES INFLAMMATOIRES

(30) Priority: 24.05.2005 EP 05011203
(43) Date of publication of application: 02.07.2008
(73) Proprietor: DSM IP Assets B.V., 6411 TE Heerlen (NL)
(72) Inventor: FOWLER, Ann, CH-4310 Rheinfelden (CH); RAEDERSTORFF, Daniel, F-68720 Flaxlanden (FR); SCHÜLER, Goede, 79591 Eimeldingen (DE); SCHWAGER, Joseph, F-68510 Uffheim (FR)
(74) Representative: Rabanus, Birgit
(86) International application number: PCT/EP2006/005005
(87) International publication number: WO 2006/125651

(56) References cited:
- WO-A-02/22621
- WO-A-95/00157
- WO-A-2004/100945
- DATABASE WPI Week 200577 Derwent Publications Ltd., London, GB; AN 2005-749412 XP002402177 & CN 1 569 848 A (INST CHINESE MATERIA MEDICA CHINA ACAD T) 26 January 2005 (2005-01-26)
- DATABASE WPI Week 200517 Derwent Publications Ltd., London, GB; AN 2005-152885 XP002402178 & CN 1 543 859 A (UNIV SECOND MILITARY MEDICAL) 10 November 2004 (2004-11-10)
- DATABASE WPI Week 198939 Derwent Publications Ltd., London, GB; AN 1989-282536 XP002402179 & JP 01 207233 A (KAKURITSU EISEI SHIKENJO) 21 August 1989 (1989-08-21)
- DATABASE WPI Week 198938 Derwent Publications Ltd., London, GB; AN 1989-274524 XP002402180 & JP 01 199958 A (TSUMURA & CO) 11 August 1989 (1989-08-11)
- PATENT ABSTRACTS OF JAPAN vol. 013, no. 238 (C-603), 5 June 1989 (1989-06-05) & JP 01 050818 A (TSUMURA & CO), 27 February 1989 (1989-02-27)
- DATABASE WPI Week 199217 Derwent Publications Ltd., London, GB; AN 1992-136751 XP002402181 & JP 04 077480 A (TSUMURA & CO) 11 March 1992 (1992-03-11)

## Description

The present invention relates to the use of compounds of the formula (I) as agents for the prevention, control and treatment of conditions requiring modulation of inflammation in mammals. In another aspect, the present invention relates to the use of compounds of the formula (I) as active ingredients in the manufacture of medicaments for the prevention, control and treatment of conditions requiring modulation of inflammation.

WO95/00157 discloses the use of sedanolide for the treatment of inflammatory conditions while WO 04/100945 discloses pharmaceutical and dietary compositions comprising phtalides. However, these documents are silent with regard to ligustilide for the prevention and/or treatment of degenerative joint diseases.

In accordance with the present invention it has been found that certain compounds modulate the biosynthesis of inflammatory mediators such as eicosanoids (prostaglandins, leukotrienes), cytokines, chemokines and nitric oxide. Therefore, such compounds are useful for the prevention, control and treatment of conditions requiring modulation of inflammation. The ability to control inflammation is essential for health. Absence of control of, or excessive and uncontrolled inflammation results in numerous diseases of which many are common diseases and conditions.

Thus, in one aspect, the present invention relates to the use of ligustilide for the treatment and prevention of inflammatory disorders.

In another aspect, the invention relates to the use of ligustilide as defined above in the manufacture of medicaments for the prevention, control and treatment of conditions requiring modulation of inflammation, particularly the treatment and prevention of inflammatory disorders.

**Table 0: List of preferred compounds used according to the present invention**

| | |
|---|---|
| 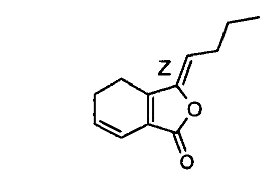 | Ligustilide |
| 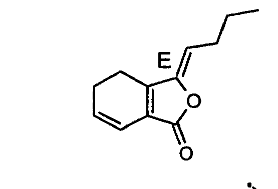 | E-Ligustilide |

: The term "ligustilide" in the context of the present invention encompasses Z-ligustilide and E-ligustilide as well as any mixture of them, especially mixtures of ≥ 90 weight-% of Z-ligustilide and ≤ 10 weight-% of E-ligustilide, based on the total weight of the mixture. Z-ligustilide is especially preferred.

For use in the present invention, ligustilide may be isolated by methods known in the art [see, e.g., Beck J.J. and Stermitz F.R., J. Natural Products, Vol. 58, No. 7, pp. 1047-1055, 1995] from various plants such as *Angelica glauca, Angelica acutiloba, Angelica sinensis, Angelicae dahuricae, Ligusticum acutilobum, Ligusticum officinale, Ligusticum sinense, Ligusticum wallichii, Cnidium officinale, Rhizoma Chuanxiong, Pleurospermum hookeri, Trachy-spermum roxburghianum, Meum athamanticum, Lomatium torreyi, Scutellaria baicalensis, Opopanax chironium, Cenolophium denudatum, Coriandrum sativuum, Silaum silaus.* The compounds used herein may also be of synthetic origin.

In a further particularly preferred embodiment of the present invention, ligustilide is used in form of a purified plant extract, e.g., from Ligusticum species, especially L. wallichii, comprising at least about 50 weight-% of ligustilide, and no more than 10 weight-% of fatty acids and triglycerides as obtainable by the process disclosed in European patent application No. 05 002333.2 and the PCT application PCT/EP2006/000648 based on it the contents of which are incorporated herein by reference.

According to an aspect of the invention disclosed in PCT patent application PCT/EP2006/000648, an extract of Ligusticum species containing less than 50 weight-% of ligustilide and more than 5 wt--% of fatty acids and glycerides is submitted to a rectification. The rectification is suitably carried out at a temperature in the range of from 130°C to 400°C, and at a pressure in the range of from 0.1 mbar to 25 mbar. In a preferred embodiment, the rectification is carried out at a heating temperature in the range of from 200°C to 230°C and at a top pressure of the rectification column in the range of from 0.1 mbar to 3 mbar. Suitably, the extract used as the starting material in this process is an extract from roots of Ligusticum species, especially dried roots from L. wallichii and is obtained by supercritical fluid extraction using, e.g., carbon dioxide. In a preferred embodiment, the extract, prior to rectification is submitted to degassing in a degassing unit. The degassing unit may be any evaporating system that allows to remove water from the extract by applying heat and reduced pressure. Conveniently, the degassing is carried out at a temperature in the range of from 120 to 180°C at 10-50 mbar.

By that process ligustilide and other compounds of formula (I) like senkyunolide, 3-n-butylphthalide, sedanolide, 3-butylidenephthalide can be enriched in the resulting distillate to over 90 % based on the weight of the distillate. In contrast to the starting material, the distillate obtained in that process smells pleasantly and shows a light yellow colour. Glycerides and free fatty acids are enriched in the distillation residue.

The rectification can be performed with all kind of evaporator types, however a preferred equipment is a wiped thin film evaporator with a short residence time, preferably not exceeding 3 minutes and low pressure drop.

The rectification column can be equipped with all kind of different column internals like trays, random or structured packings, however a preferred internal is a structured packing with a low pressure drop and a small liquid hold up. This prevents the degradation of the compounds of the formula (I) at higher temperatures and longer residence times.

A preferred rectification column set up in accordance with the process described above is equipped with a liquid side draw in the rectifying section of the column. If the resulting purified ligusticum extract is taken out of the rectification column as a liquid side draw, it leads to a higher phthalide concentration because other light boiling components compared to the phthalides can be separated with the distillate stream. The same effect can be archieved if the rectification is equipped with a divided wall column. In this case the resulting purified ligusticum extract is also taken out of the column as a side draw.

The process can be carried out batchwise and preferred in continuous mode due to the thermal instability of the phthalides. The purified extract as obtained by the process can be converted into solid formulations by conventional techniques.

Preferred examples of the process disclosed in PCT application PCT/EP2006/000648 are described in examples 11 and 12.

Ligustilide or plant extracts containing ligustilide may be used as nutraceutical compositions, i.e. as supplement to dietary compositions, i.e., food or beverages, or as compositions in dosage unit form such as pharmaceutical compositions, e.g., tablets or capsules which may further comprise pharmaceutically acceptable carriers, excipients or diluents, including, but not limited to, lubricants, colorants, wetting agents, fillers, disintegrants and flavorants.

The pharmaceutical or dietary composition may be in the form which is selected from the group consisting of fortified food or feed, beverages, tablets, granules, capsules, pastes, and effervescent formulations. The pastes may be filled into hard or soft gelatine capsules.

Ligustilide is preferably used in a concentration so that at least 0.005 mg/ kg bodyweight/ day are administered to an animal including humans.
Preferably, for use according to the present invention, an effective dose of ligustilide, for an animal including human is in the range of from 0.01 to 50 mg/ kg bodyweight/ day, more preferably in the range of from 0.1 to 25 mg/ kg bodyweight/ day, even more preferably in the range of from 0.1 to10 mg/ kg bodyweight / day, most preferably in the range of from 0.1 to 5 mg/kg body weight/day, based on the weight of the pure compounds of formula (I).

As stated above, ligustilide is useful for the prevention, control and treatment of conditions requiring modulation of inflammation Thitey can also be used as an adjunct to the treatment of a variety of diseases and disorders in which inflammation is involved.

The conditions requiring modulation of inflammation means include acute and chronic inflammatory diseases such as, degenerative joint diseases including osteoarthritis, gout and ankylosing spondylitis, tendinitis, bursitis.

So, the present invention is especially directed to the use of ligustilide as defined above (in the manufacture of a medicament/composition) for the prevention, control and treatment of conditions requiring modulation of inflammation, especially of those conditions as mentioned above.

Finally ligustilide may be used in combination with other nutraceutical compositions or therapeutic agents known to those skilled in the art for treatment or prevention of inflammatory disorders by administration prior to, simultaneously with or following the administration of ligustilide.

The following Examples are illustrative but not limitative of the invention.

### Examples

### Example 1: Soft gelatin capsule

Soft gelatin capsules are prepared by conventional procedures providing a dose of Ligustilide/ligustilide extracts of 100 mg
Other ingredients: glycerol, water, gelatine, vegetable oil

### Example 2: Hard gelatin capsule

Hard gelatin capsules are prepared by conventional procedures providing a dose of Ligustilide/ligustilide extracts of 200 mg

Other ingredients:
Fillers: lactose or cellulose or cellulose derivatives q.s
Lubricant: magnesium sterate if necessary (0.5%)

### Example 3: Tablet

Tablets are prepared by conventional procedures providing as active ingredient 50 mg of ligustilide per tablet, and as excipients microcrystalline cellulose, silicone dioxide (SiO2), magnesium stearate, crosscarmellose sodium ad 200 mg.

### Example 4: Soft drink

A Soft Drink containing a ligustilide or ligustilide extract may be prepared as follows:

### I. A Soft Drink Compound is prepared from the following ingredients:

| **1.1 Juice concentrates and water soluble flavours** | **[g]** |
|---|---|
| Orange concentrate 60.3 °Brix, 5.15% acidity | 657.99 |
| Lemon concentrate 43.5 °Brix, 32.7% acidity | 95.96 |
| Orange flavour, water soluble | 3.43 |
| Apricot flavour, water soluble | 6.71 |
| Water | 26.46 |

| **1.2 Color** | |
|---|---|
| β-Carotene 10% CWS | 0.89 |
| Water | 67.65 |

| **1.3 Acid and Antioxidant** | |
|---|---|
| Ascorbic acid | 4.11 |
| Citric acid anhydrous | 0.69 |
| Water | 43.18 |

| **1.4 Stabilizers** | |
|---|---|
| Pectin | 0.20 |
| Sodium benzoate | 2.74 |
| Water | 65.60 |

| **1.5 Oil soluble flavours** | |
|---|---|
| Orange flavour, oil soluble | 0.34 |
| Orange oil distilled | 0.34 |

| **1.6 Active ingredient** | |
|---|---|
| Ligustilide or Ligustilide extract in an amount providing 500 mg of ligustilide | |

Fruit juice concentrates and water soluble flavours are mixed without incorporation of air. The color is dissolved in deionized water. Ascorbic acid and citric acid is dissolved in water. Sodium benzoate is dissolved in water. The pectin is added unter stirring and dissolved while boiling. The solution is cooled down. Orange oil and oil soluble flavours are premixed. The active ingredient as mentioned under 1.6 is stirred into the fruit juice concentrate mixture (1.1).

In order to prepare the soft drink compound all parts 3.1.1 to 3.1.6 are mixed together before homogenising using a Turrax and then a high-pressure homogenizer (p₁ = 200 bar, p₂ = 50 bar).

### Example 5: Inhibition of inflammatory mediators

The anti-inflammatory effects of the compounds were evaluated in activated macrophages by determining the inhibition of the synthesis of nitric oxide and/or PGE₂. In order to induce an in vitro "inflammatory response", murine macrophages RAW264.7 were stimulated with lipopolysaccharide (LPS) without or with graded amounts of the test substances. Murine macrophages RAW 264.7 cells respond to LPS-stimulation by the release of substantial amounts of Prostaglandin E₂ (PGE₂) and nitric oxide (NO), which is impaired by anti-inflammatory compounds. Prostaglandins PGE₂ play a critical role in the inflammation process, while nitric oxide is a hallmark of inflammation in conditions like arthritis. Therefore, we evaluated the effect of the compounds on PGE₂ and NO production.

RAW 264.7 cells: were cultured in Dulbecco's Modified eagle Medium (DMEM) supplemented with 10% fetal calf serum (FCS), 50 units/ml penicillin, 50 µg/ml streptomycin, L-glutamine and nonessential amino acids (NEAA, Life Technologies, No. 11140). RAW cells were used between passage 10 and 50. For the experiments, cells were seeded into 6-well, 12-well or 96-well plates at 2, 1 and 0.05 mio cells per well, respectively, and used after 1 or 2 days of pre-culture. Cells were starved in complete DMEM medium containing 0.25% FCS 18 hours before the treatment. Cells were stimulated with LPS (1 µg/ml) for 24 hours in phenol-free DMEM containing 0.25% FCS. Substances to be tested were dissolved in DMSO (usually at 10 mM) and added to the culture medium concomitantly with the stimulus. Where appropriate, DMSO was added to the cell cultures to adjust the vehicle concentration, which did not exceed 0.5%. After 24 hours nitrite concentrations were measured in culture supernatants by the Griess reaction and secreted PGE₂ was determined by Enzyme immunoassay (EIA) in unstimulated and LPS-stimulated RAW264.7 cells. The compounds were simultaneously tested at different concentrations to obtain a dose response curve. The potency was evaluated by determining the concentration causing a 50% inhibition of PGE₂ or NO production and is reported as the IC₅₀. Ligustilide potently reduced the production of nitric oxide (NO) with an IC₅₀ of 12.2 ± 3.1 µM. The effects of compounds of the formula (I) on PGE2 production was also measured in the murine macrophage cell line RAW 264.7 (Table 1).

**Table 1: Inhibition of PGE₂ formation in stimulated macrophages**

| | PGE₂ Production by RAW Cells IC₅₀ (µM) |
|---|---|
| Ligustilide | 3.2 |
| N-Butylidenephthalide | 3.0 |
| 3-Butylphthalide | 3.0 |

### Example 6: Modulation of the expression levels of inflammatory genes:

THP-1 cells, a human monocyte/histiocyte cell line, were cultured in RPMI 1640 medium supplemented with 10% FCS, 50 units/ml penicillin, 50 mg/ml streptomycin, NEAA and 2x10⁻⁵ M β-mercaptoethanol. Cells were treated with 50 nM phorbol myristate acetate for 3 days. Cells were starved overnight in medium containing 0.25% FCS before being treated. Cells were stimulated with LPS (1 µg/ml) for 4 hours in phenol-free RPMI containing 0.25% FCS. Ligustilide (25µM) was added to the culture medium concomitantly with the stimulus. DMSO was added to the control cell cultures to adjust the vehicle concentration, which did not exceed 0.5%. LPS-stimutation induces the expression of a large variety of genes including those of inflammatory pathways. After 4 hours of stimulation we have evaluated the impact of ligustilide on the expression of inflammatory genes using quantitative real-time RT-PCR (reverse transcriptase polymerase chain reaction) technology (Table 2).

**Table 2: Effects of ligustilide on gene expression in THP-1 cells**

| Gene | Gene expression (in %) relative to LPS control |
|---|---|
| COX-2 | 24.1 |
| TN F-a | 50.6 |
| IL-8 | 72.4 |
| MIP-2 | 52.0 |
| MIP-3α | 67.2 |
| IL-1α | 15.2 |
| IL-6 | 52.3 |

The data of Table 2 show that ligustilide down-regulates a number of genes involved in the modulation of the inflammatory response. The cytokines TNF-α, IL-1α, IL-6, IL-8 have been implicated in acute and chronic inflammatory diseases and in osteoporosis.

### Example 7: Effects of ligustilide on carrageenan-induced paw edema in rats

The anti-inflammatory activity of the compounds was evaluated in vivo in the carrageenan-induced paw edema rat model. This model has long been used to assess the anti-inflammatory properties of agents that inhibit prostaglandins, such as nonsteroidal anti-inflammatory drugs (NSAIDs) The model causes time-dependent edema formation following carrageenan administration into the subplantar surface of a rat paw.

Twenty male Sprague-Dawley rats weighing 130 to 146 g were randomized in two groups. They were housed in a temperature (19.5-24.5°C) and relative humidity (45-65%) controlled room with a 12-h light/dark cycle, with ad libitum access to filtered tap-water and standard pelleted laboratory chow throughout the study. They were housed 5 per cage and a 5-6-day acclimatization period was observed before any testing. Animals were individually identified on the tail. Ligustilide (100 mg/kg) suspended in 1% methylcellulose (in a volume of 10 mL/kg) or vehicle alone were administered by the oral route in a coded and random order after an overnight fast. One hour later , inflammation is induced by subplantar injection of a 2 % carrageenan suspension into the right paw. The paw volume of each rat was measured in mL at the following time points: 0 h, 1.5 h, 3 h, and 4.5 h after the injection of carrageenan. The paw edema volume of each rat at each time point was expressed as the change from initial value. The anti-inflammatory effect on edema volume in treated-groups was expressed as % inhibition [(mean of vehicle-treated group paw edema volume - mean of the treated group paw edema volume)/mean of vehicle-treated group paw edema volume) x 100].

**Table 3: Pharmacological effects of ligustilide after oral administration on carrageenan-induced paw edema in rats**

| | **Paw edema volume (ml)** | | |
|---|---|---|---|
| **Time (hours)** | **vehicle-treated animals** | **Ligustilide-treated animals** | **% Inhibition of ligustilide** |
| **1.5** | 0.24 | 0.17 | 29 |
| **3** | 0.40 | 0.32 | 27 |
| **4.5** | 0.45 | 0.40 | 11 |

All data of paw edema volume are expressed in mL as Mean of 10 rats in each group.
% inhibition vs vehicle-treated group

Ligustilide (100 mg/kg) inhibited the mean paw edema volume 1.5 h, 3 h and 4.5 h after the carrageenan injection as compared to the control group treated with the vehicle.

### Example 8: Effects ligustilide in kaolin-induced arthritis in rats

Twenty male Sprague Dawley rats weighing 103 g to 132 g, were included in this study. They were housed in a temperature (19.5-24.5°C) and relative humidity (45-65%) controlled room with a 12-h light/dark cycle, with ad libitum access to filtered tap-water and standard pelleted laboratory chow, throughout the study. Upon receipt at animal facilities, they were housed 5 per cage and at least a 5-day acclimatization period were observed before any testing. Animals were individually identified on the tail. Arthritis is induced by injection of a 10 % kaolin suspension into the knee joint of the rat right hindpaw. In the vehicle-treated group, injection of kaolin suspension into the knee joint of the rat right hindpaw induced an impairment of the gait, evaluated by an increase of the gait score (score from 0 to 3). Gait of animals is used to measure the spontaneous painful behavior. Ligustilide (100 mg/kg) suspended in 1% methylcellulose (in a volume of 10 ml/kg) or vehicle alone were administered by oral route in a coded and random order, 30 min after kaolin injection. The assessment of score behavior was monitored every hour from 1.5 hours to 5.5 hours following drug dosing. The mean gait score was calculated from individual values at each time. The percentage of inhibition of the mean gait score as compared to the mean value of the control group was calculated 1.5, 2.5, 3.5, 4.5 hours and 5.5 hours after dosing.

**Table 4: Effect of ligustilide given orally on the evolution of the gait score after kaolin-induced arthritis in rats.**

| | **Gait score** | | |
|---|---|---|---|
| **Time (hours)** | **Control** | **Ligustilide** | **% improvement by ligustilide** |
| **1.5** | 0.4 | 0.3 | 25 |
| **2.5** | 0.7 | 0.4 | 43 |
| **3.5** | 1.2 | 0.6 | 50 |
| **4.5** | 1.9 | 0.9 | 53 |
| **5.5** | 2.1 | 1.3 | 38 |

The results are expressed for each group as the mean of the gait scores of 10 animals per group.

Ligustilide induced an improvement of the gait score after arthritis induction in comparison to the control group. A significant analgesic effect was observed at 3.5, 4.5 and 5.5 hours after dosing in comparison to the control group.

### Example 9: Effects of ligustilide on chondrocytes

Articulate tissues i.e. joints contain chondrocytes. Their physiological deterioration leads to the erosion of joint tissue components and thus to e.g. osteoarthritis. We have evaluated the effect of ligustilide on catabolic events in chondrocytes. SW1353 chondrosarcoma cells or normal human chondrocytes (derived from knee) were activated with interleukin-1β in the presence of graded amount of test compounds for 4 hours. RNA was then extracted from these cells and reverse-transcribed. Expression of marker genes for catabolic events like matrix metalloproteinases (MMPs) was determined by quantitative real time polymerase chain reaction (RT-PCR). As shown in Table 5 ligustilide influenced the expression level of several MMPs, which are critically involved in the destruction of extracellular matrix. Ligustilide reduced its expression and thus is supposed to prevent tissue erosion in osteoarthritic diseases. In contrast, it increased collagen mRNA levels; this suggests that it favors events that contribute to the reconstitution of the extracellular matrix.

**Table 5**

| Gene | Gene expression (in %) relative to IL-1β acti vated chondrocytes |
|---|---|
| MMP-1 | 17 |
| MMP-3 | 2 |
| MMP-9 | 33 |
| MMP-13 | 50 |
| | |
| Collagen 1 | 115 |
| Collagen 2 | 184 |

### Examples 110: Effects of ligustilide on cell adhesion to endothelial cells

Artherosclerotic lesions can develop as a consequence of endothelial dysfunction. This is reflected by altered i.e. increased adhesion of monocytes to endothelial layers. We evaluated the effect of ligustilide on adhesion of U937, a monocyte cell line, to human umbilical cord endothelial cells (HUVEC). HUVEC were stimulated with TNF-α in the absence or presence of ligustilide (25 or 50 µmol/L) for 20 hours and the adhesion of U937 was determined according to Carluccio et al. (Arterioscler Thromb Vasc Biol 2003; 23, 622-629). As shown in Table 6 adhesion was significantly impeded by ligustilide in a concentration-dependent manner. Adhesion of monocytes to endothelial layers is mediated by the expression of intercellular adhesion molecule 1 (ICAM-1). Therefore, we further analyzed the effects of ligustilide on the level of ICAM-1 mRNA in HUVEC by quantitative RT-PCR. We observed a dose-dependent reduction of ICAM-1 gene expression in these cells (Table 7. This reveals a molecular effect of ligustilide on cell adhesion events. Reduced adhesion of monocytes via reduced ICAM expression re-establishes the homeostasis of the endothelium and therefore contributes to prevention of atheroma formation.

**Table 6: Effect of ligustilide on monocyte adhesion (20 hours stimulation)**

| Treatment of HUVEC | Number of adherent cells | % reduction of adherence |
|---|---|---|
| TNF-α | 99 ± 30 | - |
| TNF- α + 25 µM ligustilide | 61 ± 25 | 39 |
| TNF- α +50 µM ligustilide | 27 ± 21 | 73 |

**Table 7: Effect of ligustilide on expression of intercellular adhesion molecule 1 (ICAM-1) mRNA (2 hours of stimulation)**

| Treatment of HUVEC | Relative ICAM-1 level (arbitrary units) | mRNA % reduction of expression |
|---|---|---|
| TNF-α | 59 ± 18 | - |
| TNF-α + 25 µM ligustilide | 25 ± 6 | 58 |
| TNF-α +50 µM ligustilide | 16 ± 1 | 73 |

### Example 11

Crude ligusticum extract, e.g. as obtained by supercritical extraction with carbon dioxide from Ligusticum roots with a total phthalide concentration of 29 weight-% ( 8.2 weight-% senkyunolide, 0.5 weight-% 3-n-butylphthalide, 1.2 weight-% sedanolide, 18.3 ligustilide, 0.6 weight-% 3-butylidenephthalide) was purified by a continuous vacuum rectification to a total phthalide concentration of 90 weight-% (26.4 weight-% senkyunolide, 1.6 weight-% 3-n-butylphthalide, 3.7 weight-% sedanolide, 56.3 weight-% ligustilide, 2.0 weight-% 3-butylidenephthalide).

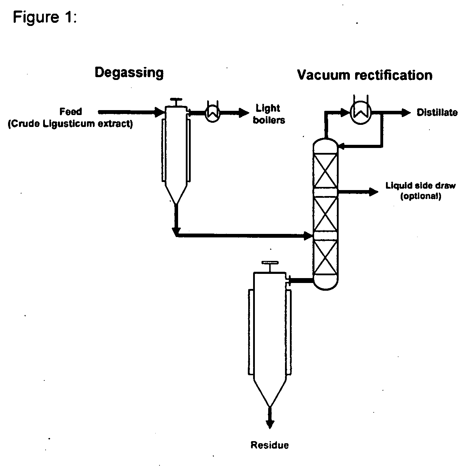

At first the crude ligusticum was degassed in order to separate the water from the crude extract. At a reduced pressure of 25 mbar and a heating temperature of 160°C approx. 1.0 % of the feed amount was evaporated. The residue comprises the crude ligusticum extract almost free off water. This material was fed continuously into the rectification setup (wiped thin film evaporator with a heating area 0.05 m2, distillation column with 1 m height structured packings) in order to concentrate the phthalides in the resulting distillate stream of the column (see Figure 1 with liquid side draw not operating). At conditions with a reduced top column pressure of 0.5 mbar and at a heating temperature of 230°C the distillate/feed-ratio was 0.33 : 1. The reflux ratio of the distillate stream was about 1. Glycerides and free fatty acids are enriched in the distillation residue. The distillate stream contains all above mentioned phthalides in a total phthalide concentration of 90 weight-%. The colour of the final purified ligusticum extract was 4.7 on Gardner scale.

### Example 12

Crude ligusticum extract, e.g., as obtained by supercritical extraction with carbon dioxide from Ligusticum roots with a total phthalide concentration of 36 weight-% ( 11.4 weight-% senkyunolide, 1.1 weight-% 3-n-butylphthalide, 1.6 weight-% sedanolide, 20.4 ligustilide, 1.3 weight-% 3-butylidenephthalide) was purified by a continuous vacuum rectification with a liquid side draw to a total phthalide concentration of 94 weight-% (29.3 weight-% senkyunolide, 3.3 weight-% 3-n-butylphthalide, 3.9 weight-% sedanolide, 53.5 weight-% ligustilide, 3.9 weight-% 3-butylidenephthalide) in the following way.

At first the crude ligusticum was degassed in order to separate the water from the crude extract. At a reduced pressure of 25 mbar and a heating temperature of 160°C approx. 1.0 % of the feed amount was evaporated. The residue comprises the crude ligusticum extract almost free off water. This material was fed continuously into the rectification setup (wiped thin film evaporator with a heating area 0.05 m2, distillation column with 1.5 m height structured packings, liquid side draw at column height 1 m from below) in order to concentrate the phthalides in the resulting liquid side stream (see Figure 1 with liquid side draw operating).

At a reduced pressure of 1 mbar and a heating temperature of 230° the feed stream was separated as follows into 36% liquid side stream, 62% residue and 2% distillate. The reflux ratio of the distillate was about 10 and the reflux ratio of the side stream was about 1. Glycerides and free fatty acids are enriched in the distillation residue and the light boiling components are enriched in the distillate. The desired phthalides are enriched in the liquid side stream with a concentration of 94%. The colour of the final purified ligusticum extract was 4.6 on Gardner scale.

## Claims

1. Ligustilide for use in the prevention, control and treatment of degenerative joint diseases in a mammal.

2. Ligustilide for use as in claim 1 wherein ligustilide is in form of a purified plant extract.

3. Ligustilide for use as in claim 1 wherein the purified plant extract is an extract from L. wallichii.

4. The use of a compound as defined in any one of claims 1 to 3 in the manufacture of a medicament for the prevention, control and treatment of degenerative joint diseases.

## Patentansprüche

1. Ligustilid zur Verwendung in der Vorbeugung, Bekämpfung und Behandlung von degenerativen Gelenkserkrankungen bei einem Säugetier.

2. Ligustilid zur Verwendung nach Anspruch 1, wobei das Ligustilid in Form eines aufgereinigten Pflanzenextrakts vorliegt.

3. Ligustilid zur Verwendung nach Anspruch 1, wobei es sich bei dem aufgereinigten Pflanzenextrakt um einen Extrakt aus L. wallichii handelt.

4. Verwendung einer Verbindung nach einem der Ansprüche 1 bis 3 in der Herstellung eines Arzneimittels für die Vorbeugung, Bekämpfung und Behandlung von degenerativen Gelenkserkrankungen.

## Revendications

1. Ligustilide destiné à être utilisé dans la prévention, le contrôle et le traitement des maladies dégénératives des articulations chez un mammifère.

2. Ligustilide destiné à être utilisé selon la revendication 1, **caractérisé en ce que** le ligustilide est sous forme d'un extrait de plante purifié.

3. Ligustilide destiné à être utilisé selon la revendication 1, **caractérisé en ce que** l'extrait de plante purifié est un extrait de L. wallichii.

4. Utilisation d'un composé tel que défini dans l'une quelconque des revendications 1 à 3, dans la fabrication d'un médicament destiné à la prévention, le contrôle et le traitement des maladies dégénératives des articulations.
